# EUROPEAN PATENT SPECIFICATION

(11) **EP 4 132 440 B1**
(45) Date of publication and mention of the grant of the patent: **10.07.2024**
(21) Application number: 21720845.3
(22) Date of filing: 15.04.2021
(51) Int. Cl.: A61F 13/00, A61F 13/02

(54) **TISSUE INTERFACE FOR NEGATIVE PRESSURE AND INSTILLATION THERAPY**
GEWEBESCHNITTSTELLE FÜR UNTERDRUCK- UND INSTILLATIONSTHERAPIE
INTERFACE TISSULAIRE POUR THÉRAPIE PAR PRESSION NÉGATIVE ET INSTILLATION

(30) Priority: 05.05.2020 US 202063020361 P
(43) Date of publication of application: 15.02.2023
(73) Proprietor: KCI Manufacturing Unlimited Company, Dublin, D01C4E0 (IE)
(72) Inventor: LOCKE, Christopher Brian, San Antonio, Texas 78265 (US)
(74) Representative: Simmons & Simmons
(86) International application number: PCT/IB2021/053133
(87) International publication number: WO 2021/224698

(56) References cited:
- WO-A1-2020/036785
- US-A1- 2015 320 434

## Description

### TECHNICAL FIELD

The invention set forth in the appended claims relates generally to tissue treatment systems and more particularly, but without limitation, to a dressing for the removal of thick exudate in a negative-pressure therapy environment.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

WO2020/036785 discloses a negative pressure wound therapy device with a modulating layer formed from an open-cell foam and a macro-column layer formed from a felted foam.

US2015/0320434 discloses a negative pressure wound therapy system with a contact layer having a walls defining a plurality of through holes.

### BRIEF SUMMARY

The invention is defined by the appended independent claim. A selection of optional features of the invention is set out in the dependent claims.

Insofar as the term embodiment is used in the following, or features are presented as being optional, this should be interpreted in such a way that the only protection sought is that of the invention claimed. Reference(s) to "embodiment(s)" throughout the description which are not under the scope of the appended claims merely represent possible exemplary executions and are not part of the present invention.

### BRIEF DESCRIPTION OF THE DRAWINGS

Figure 1 is a simplified functional block diagram of an example embodiment of a therapy system that can provide negative-pressure therapy with instillation of topical treatment solutions to a tissue site in accordance with this specification;
Figure 2 is an assembly view of an example of a dressing of Figure 1, illustrating additional details that may be associated with some embodiments of a tissue interface;
Figure 3 is a plan view illustrating additional details that may be associated with some embodiments of the tissue interface of Figure 2;
Figure 4 is a plan view illustrating additional details that may be associated with some embodiments of a hole of the contact layer of Figure 2;
Figure 5 is a plan view illustrating additional details of a portion of the contact layer of Figure 2;
Figure 6 is a plan view illustrating additional details of the tissue interface of Figure 2 in a contracted state;
Figure 7 is a sectional view of a portion of the tissue interface of Figure 2, illustrating additional details that may be associated with some embodiments;
Figure 8 is a sectional view of the tissue interface of Figure 2 during negative-pressure therapy, illustrating additional details that may be associated with some embodiments;
Figure 9 is a detail view of a portion of the tissue interface of Figure 8, illustrating additional details of the operation of the tissue interface during negative-pressure therapy; and
Figures 10-14 illustrate operational steps of a process for manufacturing the tissue interface of Figure 2, illustrating additional details that may be associated with some embodiments.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

The term "tissue site" in this context broadly refers to a wound, defect, or other treatment target located on or within tissue, including but not limited to, a surface wound, bone tissue, adipose tissue, muscle tissue, neural tissue, dermal tissue, vascular tissue, connective tissue, cartilage, tendons, or ligaments. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted. A surface wound, as used herein, is a wound on the surface of a body that is exposed to the outer surface of the body, such as injury or damage to the epidermis, dermis, and/or subcutaneous layers. Surface wounds may include ulcers or closed incisions, for example. A surface wound, as used herein, does not include wounds within an intra-abdominal cavity. A wound may include chronic, acute, traumatic, subacute, and dehisced wounds, partial-thickness burns, ulcers (such as diabetic, pressure, or venous insufficiency ulcers), flaps, and grafts, for example.

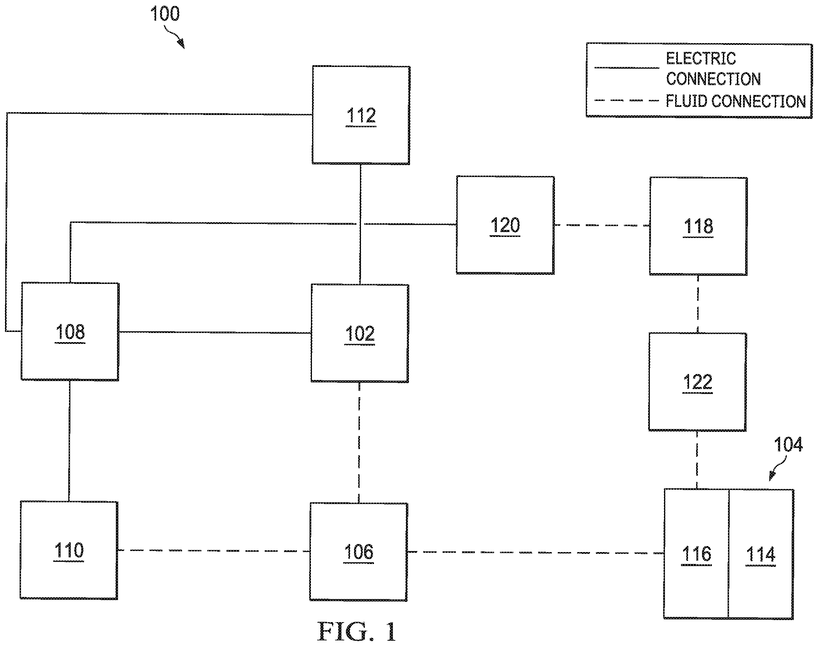

Figure 1 is a simplified functional block diagram of an example embodiment of a therapy system 100 that can provide negative-pressure therapy with instillation of topical treatment solutions to a tissue site in accordance with this specification. The therapy system 100 may include a source or supply of negative pressure, such as a negative-pressure source 102, a dressing 104, a fluid container, such as a container 106, and a regulator or controller, such as a controller 108, for example. Additionally, the therapy system 100 may include sensors to measure operating parameters and provide feedback signals to the controller 108 indicative of the operating parameters. As illustrated in Figure 1, for example, the therapy system 100 may include a pressure sensor 110, an electric sensor 112, or both, coupled to the controller 108. As illustrated in the example of Figure 1, the dressing 104 may comprise or consist essentially of a contact layer 202, a cover 116, or both in some embodiments.

The therapy system 100 may also include a source of instillation solution. For example, a solution source 118 may be fluidly coupled to the dressing 104, as illustrated in the example embodiment of Figure 1. The solution source 118 may be fluidly coupled to a positive-pressure source such as the positive-pressure source 120, a negative-pressure source such as the negative-pressure source 102, or both in some embodiments. A regulator, such as an instillation regulator 122, may also be fluidly coupled to the solution source 118 and the dressing 104 to ensure proper dosage of instillation solution (e.g. saline or sterile water) to a tissue site. For example, the instillation regulator 122 may comprise a piston that can be pneumatically actuated by the negative-pressure source 102 to draw instillation solution from the solution source during a negative-pressure interval and to instill the solution to a dressing during a venting interval. Additionally or alternatively, the controller 108 may be coupled to the negative-pressure source 102, the positive-pressure source 120, or both, to control dosage of instillation solution to a tissue site. In some embodiments, the instillation regulator 122 may also be fluidly coupled to the negative-pressure source 102 through the dressing 104, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 102 may be combined with the solution source 118, the controller 108, and other components into a therapy unit.

In general, components of the therapy system 100 may be coupled directly or indirectly. For example, the negative-pressure source 102 may be directly coupled to the container 106, and may be indirectly coupled to the dressing 104 through the container 106. Coupling may include fluid, mechanical, thermal, electrical, or chemical coupling (such as a chemical bond), or some combination of coupling in some contexts. For example, the negative-pressure source 102 may be electrically coupled to the controller 108, and may be fluidly coupled to one or more distribution components to provide a fluid path to a tissue site. In some embodiments, components may also be coupled by virtue of physical proximity, being integral to a single structure, or being formed from the same piece of material. For example, the contact layer 202 and the cover 116 may be discrete layers disposed adjacent to each other, and may be joined together in some embodiments.

A distribution component is preferably detachable, and may be disposable, reusable, or recyclable. The dressing 104 and the container 106 are illustrative of distribution components. A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 104.

A negative-pressure supply, such as the negative-pressure source 102, may be a reservoir of air at a negative pressure, or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between - 50 mm Hg (-6.7 kPa) and -300 mm Hg (-39.9 kPa).

The container 106 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 108, may be a microprocessor or computer programmed to operate one or more components of the therapy system 100, such as the negative-pressure source 102. In some embodiments, for example, the controller 108 may be a microcontroller, which generally comprises an integrated circuit containing a processor core and a memory programmed to directly or indirectly control one or more operating parameters of the therapy system 100. Operating parameters may include the power applied to the negative-pressure source 102, the pressure generated by the negative-pressure source 102, or the pressure distributed to the contact layer 202, for example. The controller 108 is also preferably configured to receive one or more input signals, such as a feedback signal, and programmed to modify one or more operating parameters based on the input signals.

Sensors, such as the pressure sensor 110 or the electric sensor 112, are generally known in the art as any apparatus operable to detect or measure a physical phenomenon or property, and generally provide a signal indicative of the phenomenon or property that is detected or measured. For example, the pressure sensor 110 and the electric sensor 112 may be configured to measure one or more operating parameters of the therapy system 100. In some embodiments, the pressure sensor 110 may be a transducer configured to measure pressure in a pneumatic pathway and convert the measurement to a signal indicative of the pressure measured. In some embodiments, the pressure sensor 110 may be a piezoresistive strain gauge. The electric sensor 112 may optionally measure operating parameters of the negative-pressure source 102, such as the voltage or current, in some embodiments. Preferably, the signals from the pressure sensor 110 and the electric sensor 112 are suitable as an input signal to the controller 108, but some signal conditioning may be appropriate. For example, the signal may need to be filtered or amplified before it can be processed by the controller 108. Typically, the signal is an electrical signal, but may be represented in other forms, such as an optical signal.

The contact layer 202 can be generally adapted to partially or fully contact a tissue site. The contact layer 202 may take many forms, and may have many sizes, shapes, or thicknesses depending on a variety of factors, such as the type of treatment being implemented or the nature and size of a tissue site. For example, the size and shape of the contact layer 202 may be adapted to the contours of deep and irregular shaped tissue sites.

In some embodiments, the cover 116 may provide a bacterial barrier and protection from physical trauma. The cover 116 may also be constructed from a material that can reduce evaporative losses and provide a fluid seal between two components or two environments, such as between a therapeutic environment and a local external environment. The cover 116 may be, for example, an elastomeric film or membrane that can provide a seal adequate to maintain a negative pressure at a tissue site for a given negative-pressure source. The cover 116 may have a high moisture-vapor transmission rate (MVTR) in some applications. For example, the MVTR may be at least about 300 g/m² per twenty-four hours in some embodiments. In some example embodiments, the cover 116 may be a polymer drape, such as a polyurethane film, that is permeable to water vapor but impermeable to liquid. Such drapes typically have a thickness in the range of about 25 microns to about 50 microns. For permeable materials, the permeability generally should be low enough that a desired negative pressure may be maintained.

The cover 116 may comprise, for example, one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Coveris Advanced Coatings of Wrexham, United Kingdom having, for example, an MVTR (inverted cup technique) of about 14400 g/m²/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; copolyester; silicones; a silicone drape; a 3M Tegaderm^{®} drape; a polyurethane (PU) drape such as one available from Avery Dennison Corporation of Glendale, California; polyether block polyamide copolymer (PEBAX), for example, from Arkema, France; INSPIRE 2327; or other appropriate material.

An attachment device may be used to attach the cover 116 to an attachment surface, such as undamaged epidermis, a gasket, or another cover. The attachment device may take many forms. For example, an attachment device may be a medically-acceptable, pressure-sensitive adhesive configured to bond the cover 116 to epidermis around a tissue site. In some embodiments, for example, some or all of the cover 116 may be coated with an adhesive, such as an acrylic adhesive, which may have a coating weight between about 25 grams per square meter (g.s.m.) and about 65 g.s.m. Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 118 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

During treatment of a tissue site, some tissue sites may not heal according to the normal medical protocol and may develop areas of necrotic tissue. Necrotic tissue may be dead tissue resulting from infection, toxins, or trauma that caused the tissue to die faster than the tissue can be removed by the normal body processes that regulate the removal of dead tissue. Sometimes, necrotic tissue may be in the form of slough, which may include a viscous liquid mass of tissue. Generally, slough is produced by bacterial and fungal infections that stimulate an inflammatory response in the tissue. Slough may be a creamy yellow color and may also be referred to as pus. Necrotic tissue may also include eschar. Eschar may be a portion of necrotic tissue that has become dehydrated and hardened. Eschar may be the result of a burn injury, gangrene, ulcers, fungal infections, spider bites, or anthrax. Eschar may be difficult to remove without the use of surgical cutting instruments.

In addition to necrotic tissue, slough, and eschar, the tissue site may include biofilms, lacerated tissue, devitalized tissue, contaminated tissue, damaged tissue, infected tissue, exudate, highly viscous exudate, fibrinous slough and/or other material that can generally be referred to as debris. The debris may inhibit the efficacy of tissue treatment and slow the healing of the tissue site. If the debris is in the tissue site, the tissue site may be treated with different processes to disrupt the debris. Examples of disruption can include softening of the debris, separation of the debris from desired tissue, such as the subcutaneous tissue, preparation of the debris for removal from the tissue site, and removal of the debris from the tissue site.

The debris can require debridement performed in an operating room. In some cases, tissue sites requiring debridement may not be life-threatening, and debridement may be considered low-priority. Low-priority cases can experience delays prior to treatment as other, more life-threatening, cases may be given priority for an operating room. As a result, low priority cases may need temporization. Temporization can include stasis of a tissue site that limits deterioration of the tissue site prior to other treatments, such as debridement, negative-pressure therapy or instillation.

When debriding, clinicians may find it difficult to define separation between healthy, vital tissue and necrotic tissue. As a result, normal debridement techniques may remove too much healthy tissue or not enough necrotic tissue. If non-viable tissue demarcation does not extend deeper than the deep dermal layer, or if the tissue site is covered by the debris, such as slough or fibrin, gentle methods to remove the debris should be considered to avoid excess damage to the tissue site.

In some debridement processes, a mechanical process is used to remove the debris. Mechanical processes may include using scalpels or other cutting tools having a sharp edge to cut away the debris from the tissue site. Other mechanical processes may use devices that can provide a stream of particles to impact the debris to remove the debris in an abrasion process, or jets of high pressure fluid to impact the debris to remove the debris using water-jet cutting or lavage. Typically, mechanical processes of debriding a tissue site may be painful and may require the application of local anesthetics. Mechanical processes also risk over removal of healthy tissue that can cause further damage to the tissue site and delay the healing process.

Debridement may also be performed with an autolytic process. For example, an autolytic process may involve using enzymes and moisture produced by a tissue site to soften and liquefy the necrotic tissue and debris. Typically, a dressing may be placed over a tissue site having debris so that fluid produced by the tissue site may remain in place, hydrating the debris. Autolytic processes can be pain-free, but autolytic processes are a slow and can take many days. Because autolytic processes are slow, autolytic processes may also involve many dressing changes. Some autolytic processes may be paired with negative-pressure therapy so that, as debris hydrates, negative pressure supplied to a tissue site may draw off the debris. In some cases, a manifold positioned at a tissue site to distribute negative-pressure across the tissue site may become blocked or clogged with debris broken down by an autolytic process. If a manifold becomes clogged, negative-pressure may not be able to remove debris, which can slow or stop the autolytic process.

Debridement may also be performed by adding enzymes or other agents to the tissue site that digest tissue. Often, strict control of the placement of the enzymes and the length of time the enzymes are in contact with a tissue site must be maintained. If enzymes are left on a tissue site for longer than needed, the enzymes may remove too much healthy tissue, contaminate the tissue site, or be carried to other areas of a patient. Once carried to other areas of a patient, the enzymes may break down undamaged tissue and cause other complications.

Some dressings or tools used for debridement may be formed from a dressing material such as foam. For example, some dressings for debridement may be manufactured using processes that involve cutting the dressing. For dressings formed from a foam, cutting of the dressing during manufacturing may make the dressing more susceptible to the shedding of particulates at the point of use. Particulates that are shed from the dressing can be left in a tissue site, potentially decreasing the efficacy of post treatment healing. Furthermore, dressings manufactured using cutting processes may be susceptible to variability in the manufacturing process. For example, a dressing a plurality of holes may have a hole depth that is not uniform. The variability of the dressing can lead to loss of efficacy of the dressing. Still further, some dressings that may be manufactured using cutting and/or tearing processes may have thinned regions or sharp edges that allow tissue in-growth at the point of use. Tissue in-growth may make it more likely that portions of the dressing remain in place following treatment, or may make it more likely that subsequent surgical procedures may be necessary to separate the tissue from the dressing.

These limitations and others may be addressed by the therapy system 100, which can provide negative-pressure therapy, instillation therapy, and disruption of debris. In some embodiments, the therapy system 100 can provide mechanical movement at a surface of the tissue site in combination with cyclic delivery and dwell of topical solutions to help solubilize debris. For example, a negative-pressure source may be fluidly coupled to a tissue site to provide negative pressure to the tissue site for negative-pressure therapy. In some embodiments, a fluid source may be fluidly coupled to a tissue site to provide therapeutic fluid to the tissue site for instillation therapy. In some embodiments, the therapy system 100 may include a contact layer positioned adjacent to a tissue site that may be used with negative-pressure therapy, instillation therapy, or both to disrupt areas of a tissue site having debris. Following the disruption of the debris, negative-pressure therapy, instillation therapy, and other processes may be used to remove the debris from a tissue site. In some embodiments, the therapy system 100 may be used in conjunction with other tissue removal and debridement techniques. For example, the therapy system 100 may be used prior to enzymatic debridement to soften the debris. In another example, mechanical debridement may be used to remove a portion of the debris at the tissue site, and the therapy system 100 may then be used to remove the remaining debris while reducing the risk of trauma to the tissue site. The therapy system 100 may also provide a process of manufacturing the dressing without cutting or tearing the dressing, minimizing the risk of shedding particulates or and the risk of tissue ingrowth.

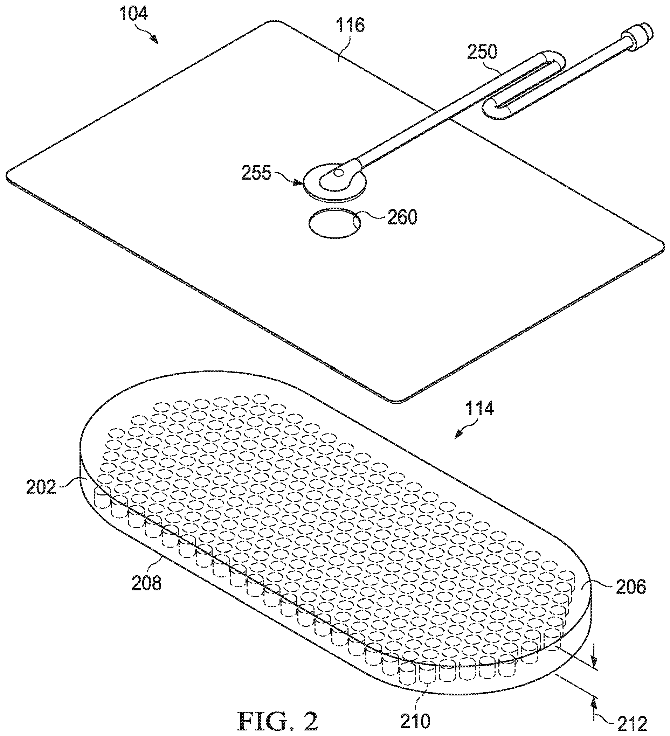

Figure 2 is an assembly view of an example of the dressing 104 of Figure 1, illustrating additional details that may be associated with some embodiments in which the contact layer 202 comprises multiple layers. In some embodiments, the contact layer 202 can include a debridement tool, such as, a contact layer 202. The contact layer 202 may have a first surface 206, a contact surface, such as a second surface 208, and a plurality of debridement cavities or blind apertures, such as a plurality of holes 210 extending into the contact layer 202 from the second surface 208 toward the first surface 206. In other embodiments, the contact layer 202 may also include a retainer layer. The retainer layer can be disposed over the contact layer 202. For example, the retainer layer may be positioned adjacent to the first surface 206 of the contact layer 202.

The contact layer 202 may have a substantially uniform thickness 212. In some embodiments, the thickness 212 may be between about 7 mm and about 32 mm. In other embodiments, the thickness 212 may be thinner or thicker than the stated range as needed for the particular application of the dressing 104. In a preferred embodiment, the thickness 212 may be about 10 mm. In some embodiments, individual portions of the contact layer 202 may have a minimal tolerance from the thickness 212. In some embodiments, the thickness 212 may have a tolerance of about 2 mm. The contact layer 202 may be flexible so that the contact layer 202 can be contoured to a surface of the tissue site.

In some embodiments, the contact layer 202 may be formed from a foam. For example, cellular foam, open-cell foam, reticulated foam, or porous tissue collections, may be used to form the contact layer 202. In some embodiments, the contact layer 202 may be a foam having pore sizes in a range of about 60 microns to about 2000 microns. In other embodiments, the contact layer 202 may be a foam having pore sizes in a range of about 400 microns to about 600 microns. The tensile strength of the contact layer 202 may vary according to needs of a prescribed therapy. For example, the tensile strength of a foam may be increased for instillation of topical treatment solutions. The 25% compression load deflection of the contact layer 202 may be at least 2.4kPa (0.35 pounds per square) inch, and the 65% compression load deflection may be at least 3kPa (0.43 pounds per square inch). In some embodiments, the tensile strength of the contact layer 202 may be at least 68.9kPa (10 pounds per square inch). The contact layer 202 may have a tear strength of at least 437.8 newtons per meter (2.5 pounds per inch). In one non-limiting example, the contact layer 202 may be an open-cell, reticulated polyurethane foam such as V.A.C. ^{®} GRANUFOAM^{™} Dressing available from Kinetic Concepts, Inc. of San Antonio, Texas; in other embodiments the contact layer 202 may be an open-cell, reticulated polyurethane foam such as a V.A.C. VERAFLO^{™} dressing, also available from Kinetic Concepts, Inc., of San Antonio, Texas. In other embodiments, the contact layer 202 may be formed of an un-reticulated open-cell foam.

In some embodiments, the contact layer 202 may be formed from a foam that is mechanically or chemically compressed, often as part of a thermoforming process, to increase the density of the foam at ambient pressure. A foam that is mechanically or chemically compressed may be referred to as a compressed foam or a felted foam. A compressed foam may be characterized by a firmness factor (FF) that is defined as a ratio of the density of a foam in a compressed state to the density of the same foam in an uncompressed state. For example, a firmness factor (FF) of 5 may refer to a compressed foam having a density at ambient pressure that is five times greater than a density of the same foam in an uncompressed state at ambient pressure. Generally a compressed or felted foam may have a firmness factor greater than 1.

Mechanically or chemically compressing a foam may reduce a thickness of the foam at ambient pressure when compared to the same foam that has not been compressed. Reducing a thickness of a foam by mechanical or chemical compression may increase a density of the foam, which may increase the firmness factor (FF) of the foam. Increasing the firmness factor (FF) of a foam may increase a stiffness of the foam in a direction that is parallel to a thickness of the foam. For example, increasing a firmness factor (FF) of the contact layer 202 may increase a stiffness of the contact layer 202 in a direction that is parallel to the thickness 212 of the contact layer 202. In some embodiments, a compressed foam may be a compressed V.A.C. ^{®} GRANUFOAM^{™} Dressing. V.A.C. ^{®} GRANUFOAM^{™} Dressing may have a density of about 0.03 grams per centimeter³ (g/cm³) in its uncompressed state. If the V.A.C.^{®} GRANUFOAM^{™} Dressing is compressed to have a firmness factor (FF) of 5, the V.A.C.^{®} GRANUFOAM^{™} Dressing may be compressed until the density of the V.A.C.^{®} GRANUFOAM^{™} Dressing is about 0.15g/cm³. V.A.C. VERAFLO^{™} dressing may also be compressed to form a compressed foam having a firmness factor (FF) up to 5. For example, V.A.C. VERAFLO^{™} Dressing, may have a density between about 1.7 pounds per foot³ (lb/ft³) or 0.027 grams per centimeter³ (g/cm³) and about 2.1 lb/ft³ or 0.034 g/cm³. If the V.A.C. VERAFLO^{™} Dressing is compressed to have a firmness factor (FF) of 5, the V.A.C. VERAFLO^{™} Dressing may be compressed until the density of the V.A.C. VERAFLO^{™} Dressing is between about 0.135 g/cm³and about 0.17 g/cm³. In some embodiments, the contact layer 202 may have a thickness between about 14 mm and about 64 mm, and more specifically, about 32 mm at ambient pressure. In an exemplary embodiment, if the thickness 212 of the contact layer 202 is about 32 mm, and the contact layer 202 is positioned within the sealed environment and subjected to negative pressure of about -115 mm Hg to about -135 mm Hg, the thickness 212 of the contact layer 202 may be between about 4 mm and about 20 mm and, generally, greater than about 12 mm.

Generally, if a compressed foam is subjected to negative pressure, the compressed foam exhibits less deformation than a similar uncompressed foam. If the contact layer 202 is formed of a compressed foam, the thickness 212 of the contact layer 202 may deform less than if the contact layer 202 is formed of a comparable uncompressed foam. The decrease in deformation may be caused by the increased stiffness as reflected by the firmness factor (FF). If subjected to the stress of negative pressure, the contact layer 202 that is formed of compressed foam may flatten less than the contact layer 202 that is formed from uncompressed foam. Consequently, if negative pressure is applied to the contact layer 202, the stiffness of the contact layer 202 in the direction parallel to the thickness 212 of the contact layer 202 allows the contact layer 202 to be more compliant or compressible in other directions, e.g., a direction perpendicular to the thickness 212. The foam material used to form a compressed foam may be either hydrophobic or hydrophilic. The foam material used to form a compressed foam may also be either reticulated or un-reticulated. The pore size of a foam material may vary according to needs of the contact layer 202 and the amount of compression of the foam. For example, in some embodiments, an uncompressed foam may have pore sizes in a range of about 400 microns to about 600 microns. If the same foam is compressed, the pore sizes may be smaller than when the foam is in its uncompressed state.

In some embodiments, the contact layer 202 may further comprise one or more retainer layers disposed over the contact layer 202. The retainer layer may be formed form an open-cell reticulated foam. The retainer layer may substantially fill the tissue site between the first surface 206 of the contact layer 202 and an edge of the tissue site surrounded by undamaged epidermis.

As illustrated in the example of Figure 2, in some embodiments, the dressing 104 may include a fluid conductor 250 and a dressing interface 255. As shown in the example of Figure 2, the fluid conductor 250 may be a flexible tube, which can be fluidly coupled on one end to the dressing interface 255. The dressing interface 255 may be an elbow connector, as shown in the example of Figure 2, which can be placed over an aperture 260 in the cover 116 to provide a fluid path between the fluid conductor 250 and the contact layer 202. In some embodiments, the contact layer 202 may be provided as a portion of an assembly forming the dressing 104. In other embodiments, the contact layer 202 may be provided separately from the cover 116, the fluid conductor 250, and the dressing interface 255 for assembly of the dressing 104 at the point of use.

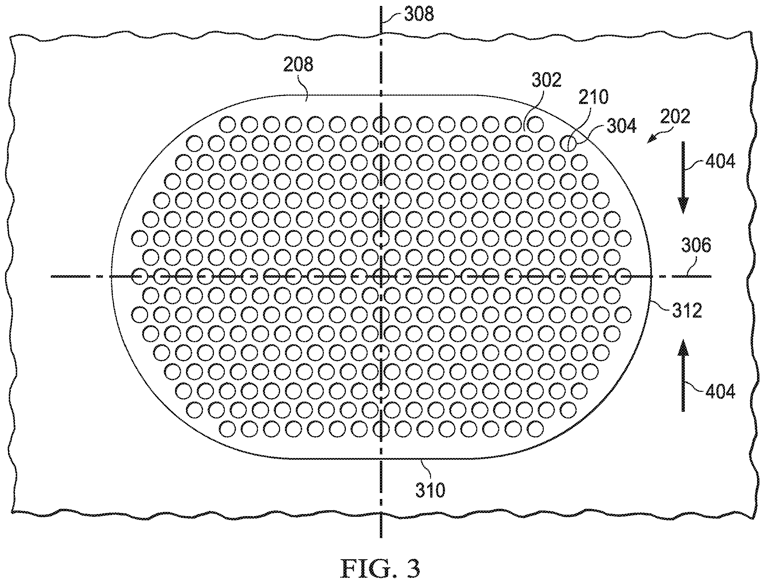

Figure 3 is a plan view, illustrating additional details that may be associated with some embodiments of the contact layer 202. In some embodiments, the holes 210 can be distributed about the second surface 208 of the contact layer 202. The holes 210 can be evenly distributed. In other embodiments, the holes 210 may be preferentially disposed in a portion of the contact layer 202. For example, the contact layer 202 may include the plurality of holes 210 or other perforations extending into the contact layer 202 to form walls 302. In some embodiments, an exterior surface of the walls 302 may be parallel to sides of the contact layer 202. In other embodiments, an interior surface of the walls 302 may be generally perpendicular to the second surface 208 of the contact layer 202. Generally, the exterior surface or surfaces of the walls 302 may be coincident with the second surface 208. The interior surface or surfaces of the walls 302 may form a perimeter 304 of each hole 210. In some embodiments, the holes 210 may have a circular shape as shown. In some embodiments, the holes 210 may have average effective diameters between about 5 mm and about 20 mm, and in some embodiments, the average effective diameters of the holes 210 may be about 10 mm.

In some embodiments, the contact layer 202 may have a first orientation line 306 and a second orientation line 308 that is perpendicular to the first orientation line 306. The first orientation line 306 and the second orientation line 308 may be lines of symmetry of the contact layer 202. A line of symmetry may be, for example, an imaginary line across the second surface 208 or the first surface 206 of the contact layer 202 defining a fold line such that if the contact layer 202 is folded on the line of symmetry, the holes 210 and the walls 302 on each side would be coincidentally aligned. Generally, the first orientation line 306 and the second orientation line 308 aid in the description of the contact layer 202. In some embodiments, the first orientation line 306 and the second orientation line 308 may be used to refer to the desired directions of contraction of the contact layer 202. For example, the desired direction of contraction may be parallel to the second orientation line 308 and perpendicular to the first orientation line 306. In other embodiments, the desired direction of contraction may be parallel to the first orientation line 306 and perpendicular to the second orientation line 308. In still other embodiments, the desired direction of contraction may be at a non-perpendicular angle to both the first orientation line 306 and the second orientation line 308. In other embodiments, the contact layer 202 may not have a desired direction of contraction.

Generally, the contact layer 202 may be placed at the tissue site so that the second orientation line 308 extends across debris located at the tissue site. Although the contact layer 202 is shown as having a generally ovoid shape including longitudinal edges 310 and circular edges 312, the contact layer 202 may have other shapes. For example, the contact layer 202 may have a rectangular, diamond, square, circular, triangular, or amorphous shape. In some embodiments, the shape of the contact layer 202 may be selected to accommodate the type of tissue site being treated. For example, the contact layer 202 may have an oval or circular shape to accommodate an oval or circular tissue site. The contact layer 202 may be sizeable. For example, the contact layer 202 may be cut, torn, or otherwise separated into portions to permit the contact layer 202 to be diminished in size for smaller tissue sites. In some embodiments, the first orientation line 306 may be parallel to the longitudinal edges 310.

Similarly, the contact layer 202 may include the plurality of holes 210 or other perforations extending into the contact layer 202 to form walls. In some embodiments, an exterior surface of the walls 302 may be parallel to sides of the contact layer 202. In other embodiments, an interior surface of the walls 302 may be generally perpendicular to the second surface 208 of the contact layer 202. Generally, the exterior surface or surfaces of the walls may be coincident with the second surface 208. The interior surface or surfaces of the walls may form a perimeter 304 of each hole 210 and may connect to the first surface 206. In some embodiments, the holes 210 may have a circular shape as shown. In some embodiments, the holes 210 may have average effective diameters between about 5 mm and about 20 mm, and in some embodiments, the average effective diameters of the holes 210 may be about 10 mm. In some embodiments, the holes 210 may be blind holes. For example, the holes 210 may have a depth that is less than the thickness 212 of the contact layer 202. For example, the holes 210 may have a depth between about 1 mm to about 10 mm, and more specifically, between about 5 mm and about 8 mm at ambient pressure.

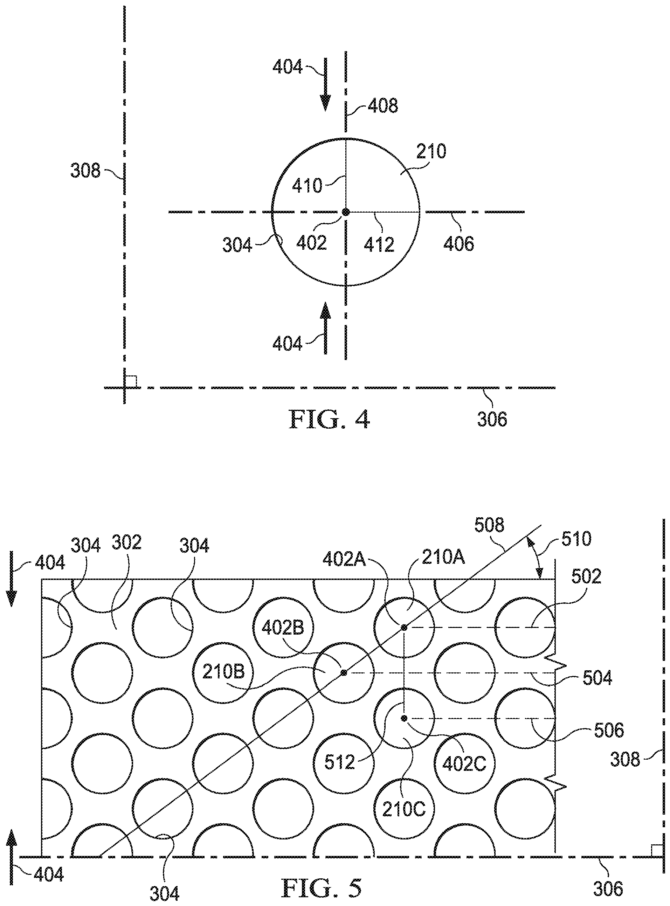

Figure 4 is a plan view illustrating additional details that may be associated with some embodiments of a hole 210 of Figure 3. In Figure 4, a single hole 210 having a circular shape is shown. The hole 210 may include a center 402 and the perimeter 304. The hole 210 may have a perforation shape factor (PSF). The perforation shape factor (PSF) may represent an orientation of the hole 210 relative to the first orientation line 306 and the second orientation line 308. Generally, the perforation shape factor (PSF) is a ratio of ½ a maximum length of the hole 210 that is parallel to the desired direction of contraction to ½ a maximum length of the hole 210 that is perpendicular to the desired direction of contraction. For descriptive purposes, the desired direction of contraction is parallel to the second orientation line 308. The desired direction of contraction may be indicated by a lateral force 404. For reference, the hole 210 may have an X-axis 406 extending through the center 402 parallel to the first orientation line 306, and a Y-axis 408 extending through the center 402 parallel to the second orientation line 308. The perforation shape factor (PSF) of the hole 210 may be defined as a ratio of a line segment 410 on the Y-axis 408 extending from the center 402 to the perimeter 304 of the hole 210, to a line segment 412 on the X-axis 406 extending from the center 402 to the perimeter 304 of the hole 210. If a length of the line segment 410 is 2.5 mm and the length of the line segment 412 is 2.5 mm, the perforation shape factor (PSF) would be 1. In other embodiments, the holes 210 may have other shapes and orientations, for example, oval, hexagonal, square, triangular, or amorphous or irregular and be oriented relative to the first orientation line 306 and the second orientation line 308 so that the perforation shape factor (PSF) may range from about 0.5 to about 1.10.

Figure 5 is a plan view illustrating additional details of the plurality of holes 210 of the contact layer 202 of Figure 3. As illustrated in Figure 5, the contact layer 202 may include the plurality of holes 210 aligned in parallel rows to form an array. The array of holes 210 may include a first row 502 of the holes 210, a second row 504 of the holes 210, and a third row 506 of the holes 210. In some embodiments, a width of the wall 302 between the perimeter 304 of adjacent holes 210 in a row, such as the first row 502, may be about 5 mm. The centers 402 of the holes 210 in adjacent rows, for example, the first row 502 and the second row 504, may be characterized by being offset from the second orientation line 308 along the first orientation line 306. In some embodiments, a line connecting the centers of adjacent rows may form a strut angle (SA) with the first orientation line 306. For example, a first hole 210A in the first row 502 may have a center 402A, and a second hole 210B in the second row 504 may have a center 402B. A strut line 508 may connect the center 402A with the center 402B. The strut line 508 may form an angle 510 with the first orientation line 306. The angle 510 may be the strut angle (SA) of the contact layer 202. In some embodiments, the strut angle (SA) may be less than about 90°. In other embodiments, the strut angle (SA) may be between about 30° and about 70° relative to the first orientation line 306. In other embodiments, the strut angle (SA) may be about 66° from the first orientation line 306. Generally, as the strut angle (SA) decreases, a stiffness of the contact layer 202 in a direction parallel to the first orientation line 306 may increase. Increasing the stiffness of the contact layer 202 parallel to the first orientation line 306 may increase the compressibility of the contact layer 202 perpendicular to the first orientation line 306. Consequently, if negative pressure is applied to the contact layer 202, the contact layer 202 may be more compliant or compressible in a direction perpendicular to the first orientation line 306. By increasing the compressibility of the contact layer 202 in a direction perpendicular to the first orientation line 306, the contact layer 202 may collapse to apply the lateral force 404 to the tissue site as described in more detail below.

In some embodiments, the centers 402 of the holes 210 in alternating rows, for example, the center 402A of the first hole 210A in the first row 502 and a center 402C of a hole 210C in the third row 506, may be spaced from each other parallel to the second orientation line 308 by a length 512. In some embodiments, the length 512 may be greater than an effective diameter of the hole 210. If the centers 402 of holes 210 in alternating rows are separated by the length 512, the exterior surface of the walls 302 parallel to the first orientation line 306 may be considered continuous. Generally, the exterior surface of the walls 302 may be continuous if the exterior surface of the walls 302 do not have any discontinuities or breaks between holes 210. In some embodiments, the length 512 may be between about 7 mm and about 25 mm.

Regardless of the shape of the holes 210, the holes 210 in the contact layer 202 may leave void spaces in the contact layer 202 and on the second surface 208 and the first surface 206 of the contact layer 202 so that only the exterior surface of the walls 302 of the contact layer 202 remain with a surface available to contact the tissue site. It may be desirable to minimize the exterior surface of the walls 302 so that the holes 210 may collapse, causing the contact layer 202 to collapse and generate the lateral force 404 in a direction perpendicular to the first orientation line 306. However, it may also be desirable not to minimize the exterior surface of the walls 302 so much that the contact layer 202 becomes too fragile for sustaining the application of a negative pressure. The void space percentage (VS) of the holes 210 may be equal to the percentage of the volume or surface area of the void spaces of the second surface 208 created by the holes 210 to the total volume or surface area of the second surface 208 of the contact layer 202. In some embodiments, the void space percentage (VS) may be between about 40% and about 75%. In other embodiments, the void space percentage (VS) may be about 55%. The organization of the holes 210 can also impact the void space percentage (VS), influencing the total surface area of the contact layer 202 that may contact the tissue site. In some embodiments, the longitudinal edge 310 and the circular edge 312 of the contact layer 202 may be discontinuous. An edge may be discontinuous where the holes 210 overlap an edge causing the edge to have a non-linear profile. A discontinuous edge may reduce the disruption of keratinocyte migration and enhance re-epithelialization while negative pressure is applied to the dressing 104.

In some embodiments, sequentially decreasing diameters of the holes 210 in subsequent applications of the contact layer 202 may aid in fine tuning a level of tissue disruption to the debris during the treatment of the tissue site. The diameter of the holes 210 can also influence fluid movement in the contact layer 202 and the dressing 104. For example, the contact layer 202 can channel fluid in the dressing 104 toward the holes 210 to aid in the disruption of the debris on the tissue site. Variation of the diameters of the holes 210 can vary how fluid is moved through the dressing 104 with respect to both the removal of fluid and the application of negative pressure. In some embodiments, the diameter of the holes 210 is between about 5 mm and about 20 mm and, more specifically, about 10 mm.

An effective diameter of a non-circular area is defined as a diameter of a circular area having the same surface area as the non-circular area. In some embodiments, each hole 210 may have an effective diameter of about 3.5 mm. In other embodiments, each hole 210 may have an effective diameter between about 5 mm and about 20 mm. The effective diameter of the holes 210 should be distinguished from the porosity of the material forming the walls 302 of the contact layer 202. Generally, an effective diameter of the holes 210 is an order of magnitude larger than the effective diameter of the pores of a material forming the contact layer 202. For example, the effective diameter of the holes 210 may be larger than about 1 mm, while the walls 302 may be formed from V.A.C. ^{®} GRANUFOAM^{™} Dressing having a pore size less than about 600 microns. In some embodiments, the pores of the walls 302 may not create openings that extend all the way through the material. Generally, the holes 210 do not include pores formed by the foam formation process, and the holes 210 may have an average effective diameter that is greater than ten times an average effective diameter of pores of a material.

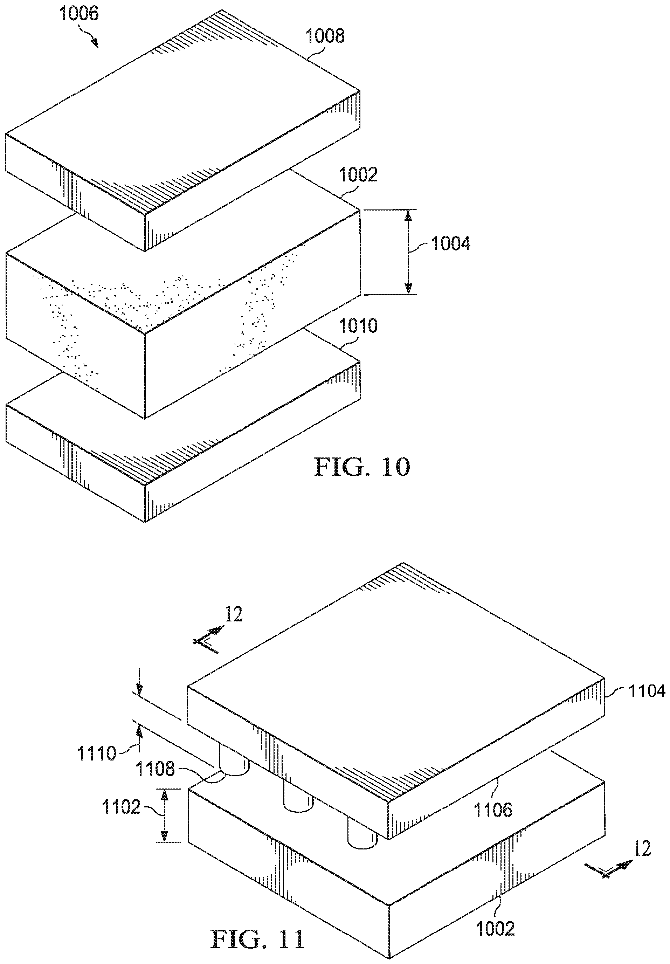

Referring now to both Figure 3 and Figure 5, the holes 210 may form a pattern depending on the geometry of the holes 210 and the alignment of the holes 210 between adjacent and alternating rows in the contact layer 202 with respect to the first orientation line 306. If the contact layer 202 is subjected to negative pressure, the holes 210 of the contact layer 202 may contract. As used herein, contraction can refer to both vertical compression of a body parallel to a thickness of the body, such as the contact layer 202, and lateral compression of a body perpendicular to a thickness of the body, such as the contact layer 202. In some embodiments the void space percentage (VS), the perforation shape factor (PSF), and the strut angle (SA) may cause the contact layer 202 to contract along the second orientation line 308 perpendicular to the first orientation line 306 as shown in more detail in Figure 10.

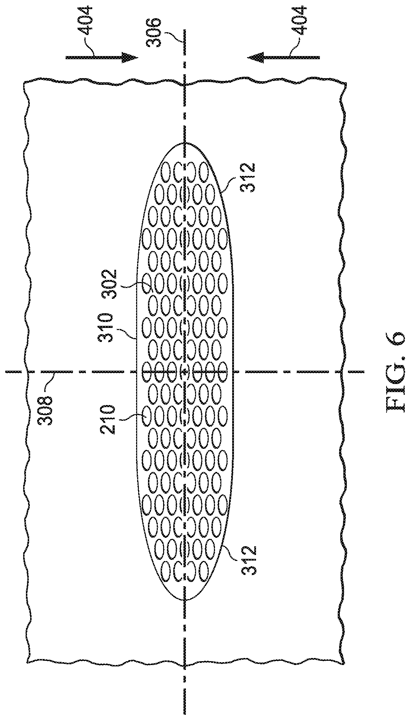

Figure 6 is a plan view illustrating additional details of the contact layer 202 of Figure 3 in a contracted state. If the contact layer 202 is positioned on the tissue site, the contact layer 202 may generate the lateral force 404 along the second orientation line 308, contracting the contact layer 202, as shown in more detail in Figure 6. In some embodiments, the holes 210 may be circular, have a strut angle (SA) of approximately 37°, a void space percentage (VS) of about 54%, a firmness factor (FF) of about 5, a perforation shape factor (PSF) of about 1, and a diameter of about 5 mm. If the contact layer 202 is subjected to a negative pressure of about -125 mm Hg, the lateral force 404 generated by the contact layer 202 is approximately 11.9 N. If the diameter of the holes 210 of the contact layer 202 is increased to about 20 mm, the void space percentage (VS) changed to about 52%, the strut angle (SA) changed to about 52°, and the perforation shape factor (PSF) and the firmness factor (FF) remain the same, the lateral force 404 is decreased to about 6.5 N. In other embodiments, the holes 210 may be hexagonal, have a strut angle (SA) of approximately 66°, a void space percentage (VS) of about 55%, a firmness factor (FF) of about 5, a perforation shape factor (PSF) of about 1.07, and an effective diameter of about 5 mm. If the contact layer 202 is subjected to a negative pressure of about -125 mm Hg, the lateral force 404 generated by the contact layer 202 is approximately 13.3 N. If the effective diameter of the holes 210 of the contact layer 202 is increased to 10 mm, the lateral force 404 is decreased to about 7.5 N.

As illustrated in Figure 6, the contact layer 202 is in the second position, or contracted position, as indicated by the lateral force 404. In operation, negative pressure is supplied to the sealed environment with the negative-pressure source 102. In response to the supply of negative pressure, the contact layer 202 contracts from the relaxed position illustrated in Figure 3 to the contracted position illustrated in Figure 6. In some embodiments, the thickness 212 of the contact layer 202 remains substantially the same. When the negative pressure is removed, for example, by venting the negative pressure from the sealed space, the contact layer 202 expands back to the relaxed position. If the contact layer 202 is cycled between the contracted and relaxed positions of Figure 3 and Figure 6, respectively, the second surface 208 of the contact layer 202 may disrupt the debris on the tissue site by rubbing the debris from the tissue site. The edges of the holes 210 formed by the second surface 208 and the interior surfaces or transverse surfaces of the walls 302 can form cutting edges that can disrupt the debris in the tissue site, allowing the debris to exit through the holes 210. In some embodiments, the cutting edges are defined by the perimeter 304 where each hole 210 intersects the second surface 208.

In some embodiments, the material, the void space percentage (VS), the firmness factor, the strut angle, the hole shape, the perforation shape factor (PSF), and the hole diameter may be selected to increase compression or collapse of the contact layer 202 in a lateral direction, as shown by the lateral force 404, by forming weaker walls 302. Conversely, the factors may be selected to decrease compression or collapse of the contact layer 202 in a lateral direction, as shown by the lateral force 404, by forming stronger walls 302. Similarly, the factors described herein can be selected to decrease or increase the compression or collapse of the contact layer 202 perpendicular to the lateral force 404.

In some embodiments, the therapy system 100 may provide cyclic therapy. Cyclic therapy may alternately apply negative pressure to and vent negative pressure from a sealed space or sealed environment containing the contact layer 202. In some embodiments, negative pressure may be supplied to the tissue site until the pressure in the sealed environment reaches a predetermined therapy pressure. If negative pressure is supplied to the sealed environment, the debris and the subcutaneous tissue underlying the debris may be drawn into the holes 210. In some embodiments, the sealed environment may remain at the therapy pressure for a predetermined therapy period such as, for example, about 10 minutes. In other embodiments, the therapy period may be longer or shorter as needed to supply appropriate negative-pressure therapy to the tissue site.

Following the therapy period, the sealed environment may be vented. For example, the negative-pressure source 102 may fluidly couple the sealed environment to the atmosphere (not shown), allowing the sealed environment to return to ambient pressure. In some embodiments, the negative-pressure source 102 may vent the sealed environment for about 1 minute. In other embodiments, the negative-pressure source 102 may vent the sealed environment for longer or shorter periods. After venting of the sealed environment, the negative-pressure source 102 may be operated to begin another negative-pressure therapy cycle.

In some embodiments, instillation therapy may be combined with negative-pressure therapy. For example, following the therapy period of negative-pressure therapy, the solution source 118 may operate to provide fluid to the sealed environment. In some embodiments, the solution source 118 may provide fluid while the negative-pressure source 102 vents the sealed environment. For example, the positive-pressure source 120 may be configured to move instillation fluid from the solution source 118 to the sealed environment. In some embodiments, the solution source 118 may not have a pump and may operate using a gravity feed system. In other embodiments, the negative-pressure source 102 may not vent the sealed environment. Instead, the negative pressure in the sealed environment is used to draw instillation fluid from the solution source 118 into the sealed environment.

In some embodiments, the solution source 118 may provide a volume of fluid to the sealed environment. In some embodiments, the volume of fluid may be the same as a volume of the sealed environment. In other embodiments, the volume of fluid may be smaller or larger than the sealed environment as needed to appropriately apply instillation therapy. Instilling of the tissue site may raise a pressure in the sealed environment to a pressure greater than the ambient pressure, for example to between about 0 mm Hg and about 15 mm Hg and, more specifically, about 5 mm Hg. In some embodiments, the fluid provided by the solution source 118 may remain in the sealed environment for a dwell time. In some embodiments, the dwell time is about 5 minutes. In other embodiments, the dwell time may be longer or shorter as needed to appropriately administer instillation therapy to the tissue site. For example, the dwell time may be zero.

At the conclusion of the dwell time, the negative-pressure source 102 may be operated to draw the instillation fluid into the container, completing a cycle of therapy. As the instillation fluid is removed from the sealed environment with negative pressure, negative pressure may also be supplied to the sealed environment, starting another cycle of therapy.

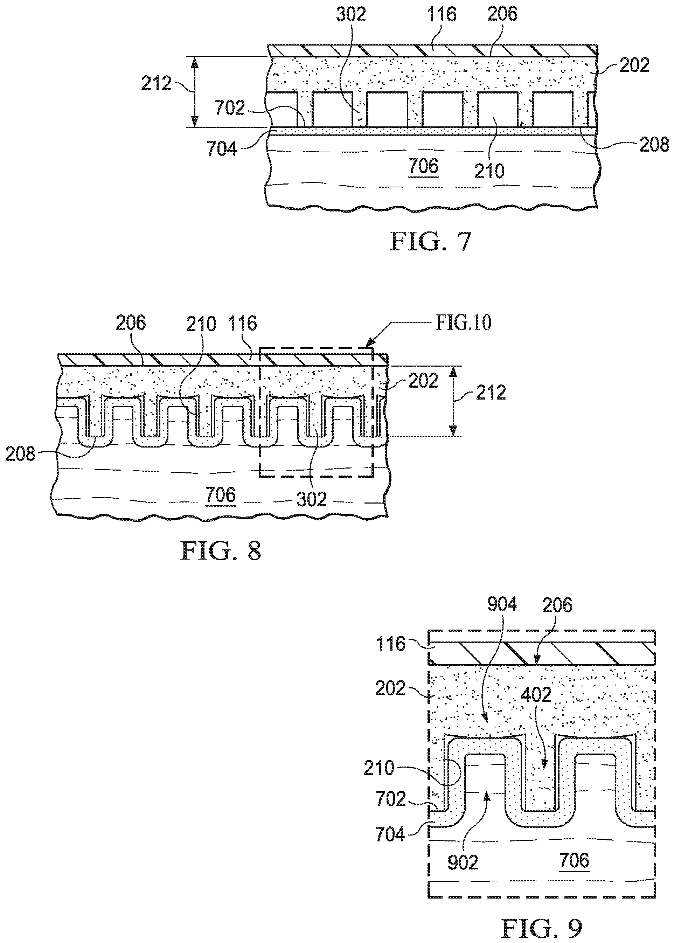

Figure 7 is a sectional view of a portion of the contact layer 202 and the contact layer 202, illustrating additional details that may be associated with some embodiments. The contact layer 202 may be placed at a tissue site 702 having debris 704 covering subcutaneous tissue 706. For example, a clinician may place the contact layer 202 having the contact layer 202 and the contact layer 202 at the tissue site 702. In some embodiments, the contact layer 202 may be packaged in a sterile container that the clinician may open and remove. The contact layer 202 having the contact layer 202 may be removed as a single piece for placement at the tissue site 702.

In some embodiments, the contact layer 202 may have a length and width that is greater than an opening of the tissue site 702. The contact layer 202 may be sized to permit the contact layer 202 to be passed through the opening of the tissue site 702 to be placed adjacent to the debris 704. Sizing can include removing a portion of the contact layer 202, for example, by cutting, tearing, melting, dissolving, vaporizing, or otherwise separating a portion of the contact layer 202 from remaining portions of the contact layer 202. Following sizing and placement of the contact layer 202 at the tissue site 702, the cover 116 may be placed over the contact layer 202 to provide a sealed environment for the application of negative-pressure therapy or instillation therapy. As shown in Figure 7, the contact layer 202 may have the thickness 212 if the pressure in the sealed environment is about an ambient pressure. In some embodiments, the thickness 212 may be about 10 mm.

Figure 8 is a sectional view of a portion of the dressing 104 during negative-pressure therapy, illustrating additional details that may be associated with some embodiments. For example, Figure 8 may illustrate a moment in time where a pressure in the sealed environment may be about - 125 mm Hg of negative pressure. In some embodiments, the contact layer 202 may be a felted foam. In response to the application of negative pressure, the contact layer 202 may not compress or compress minimally so that the thickness 212 remains substantially the same. In some embodiments, the thickness 212 of the contact layer 202 during negative-pressure therapy may be slightly less than the thickness 212 of the contact layer 202 if the pressure in the sealed environment is about the ambient pressure.

In some embodiments, negative pressure in the sealed environment can generate concentrated stresses in the contact layer 202 adjacent to the holes 210 in the contact layer 202. The concentrated stresses can cause macro-deformation of the contact layer 202 that draws portions of the contact layer 202 overlaying the holes 210 into the holes 210. Similarly, negative pressure in the sealed environment can generate concentrated stresses in the debris 704 adjacent to the holes 210 in the contact layer 202. The concentrated stresses can cause macro-deformations of the debris 704 and the subcutaneous tissue 706 that draws portions of the debris 704 and the subcutaneous tissue 706 into the holes 210.

Figure 9 is a detail view of the contact layer 202, illustrating additional details of the operation of the contact layer 202 during negative-pressure therapy. The holes 210 of the contact layer 202 may create macro-pressure points in portions of the debris 704, and the subcutaneous tissue 706 that are in contact with the second surface 208 of the contact layer 202, causing tissue puckering and nodules 902 in the debris 704 and the subcutaneous tissue 706.

A height of the nodules 902 over the surrounding tissue may be selected to maximize disruption of debris 704 and minimize damage to subcutaneous tissue 706 or other desired tissue. Generally, the pressure in the sealed environment can exert a force that is proportional to the area over which the pressure is applied. At the holes 210 of the contact layer 202, the force may be concentrated as the resistance to the application of the pressure is less than in the walls 302 of the contact layer 202. In response to the force generated by the pressure at the holes 210, the debris and the subcutaneous tissue 706 that forms the nodules 902 may be drawn into the holes 210 until the force applied by the pressure is equalized by the reactive force of the debris 704, and the subcutaneous tissue 706. In some embodiments where the negative pressure in the sealed environment may cause tearing, the depth of the holes 210 may be selected to limit the height of the nodules 902 over the surrounding tissue. In some embodiments, the height of the nodules 902 may be limited to a height that is less than the depth of the holes 210. In an exemplary embodiment, the depth of the holes 210 may be about 8 mm. During the application of negative pressure, the height of the nodules 902 may be limited to about 2 mm to about 8 mm. By controlling the height of the nodules 902 by controlling the depth of the holes 210, the aggressiveness of disruption to the debris 704 and tearing can be controlled.

In some embodiments, the height of the nodules 902 can also be controlled by controlling an expected compression of the contact layer 202 during negative-pressure therapy. For example, the contact layer 202 may have a thickness 212 of about 16 mm. If the contact layer 202 is formed from a compressed foam, the firmness factor of the contact layer 202 may be higher; however, the contact layer 202 may still reduce in thickness in response to negative pressure in the sealed environment. In one embodiment, application of negative pressure of between about -50 mm Hg and about -350 mm Hg, between about -100 mm Hg and about -250 mm Hg and, more specifically, about - 125 mm Hg in the sealed environment may reduce the thickness 212 of the contact layer 202 from about 16 mm to about 6 mm. The height of the nodules 902 may be limited to be no greater than the depth of the holes 210 during negative-pressure therapy, for example, about 3 mm. By controlling the height of the nodules 902, the forces applied to the debris 704 by the contact layer 202 can be adjusted and the degree that the debris 704 is stretched can be varied.

In some embodiments, the formation of the nodules 902 can cause the debris 704 to remain in contact with a contact layer 202 during negative pressure therapy. For example, the nodules 902 may contact the sidewalls of the holes 210 of the contact layer 202. In some embodiments, formation of the nodules 902 may lift debris 704 and particulates off of the surrounding tissue, operating in a piston-like manner to move debris 704 toward the contact layer 202 and out of the sealed environment.

Portions of the contact layer 202 overlaying the holes 210 may be drawn into the holes 210 to form bosses 904. The bosses 904 may have a shape that corresponds to the holes 210. A height of the bosses 904 may be dependent on the pressure of the negative pressure in the sealed environment, the area of the holes 210, and the firmness factor of the contact layer 202.

In some embodiments, the contact layer 202 may limit the height of the nodules 902 to the depth of the holes 210 under negative pressure. In other embodiments, the bosses 904 of the contact layer 202 may limit the height of the nodules 902 to a height that is less than the depth of the holes 210. By controlling the firmness factor of the contact layer 202, the height of the bosses 904 can be controlled. The height of the nodules 902 can be limited to the difference of the depth of the holes 210 and the height of the bosses 904. In some embodiments, the height of the bosses 904 can vary from zero to several millimeters as the firmness factor of the contact layer 202 decreases. In an exemplary embodiment, the thickness 212 of the contact layer 202 may be about 16 mm. During the application of negative pressure, the bosses 904 may have a height between about 2 mm to about 3 mm, limiting the height of the nodules 902 by about 2 mm to about 3 mm. By controlling the height of the nodules 902 by controlling the depth of the holes 210, the firmness factor of the contact layer 202, or both, the aggressiveness of disruption to the debris 704 and tearing can be controlled.

In response to the return of the sealed environment to ambient pressure by venting the sealed environment, the nodules 902 and the bosses 904 may leave the holes 210, returning to the position shown in Figure 7. In some embodiments, repeated application of negative-pressure therapy and instillation therapy while the contact layer 202 is disposed over the debris 704 may disrupt the debris 704, allowing the debris 704 to be removed during dressing changes. In other embodiments, the contact layer 202 may disrupt the debris 704 so that the debris 704 can be removed by negative pressure. In still other embodiments, the contact layer 202 may disrupt the debris 704, aiding removal of the debris 704 during debridement processes. With each cycle of therapy, the contact layer 202 may form nodules 902 in the debris 704. The formation of the nodules 902 and release of the nodules 902 by the contact layer 202 during therapy may disrupt the debris. With each subsequent cycle of therapy, disruption of the debris 704 can be increased.

Disruption of the debris 704 can be caused, at least in part, by the concentrated forces applied to the debris 704 by the holes 210 and the walls 302 of the contact layer 202. The forces applied to the debris 704 can be a function of the negative pressure supplied to the sealed environment and the area of each hole 210. For example, if the negative pressure supplied to the sealed environment is about -125 mm Hg and the diameter of each hole 210 is about 5 mm, the force applied at each hole 210 is about 0.07 lbs. If the diameter of each hole 210 is increased to about 8 mm, the force applied at each hole 210 can increase up to 6 times. Generally, the relationship between the diameter of each hole 210 and the applied force at each hole 210 is not linear and can increase exponentially with an increase in diameter.

In some embodiments, the negative pressure applied by the negative-pressure source 102 may be cycled rapidly. For example, negative pressure may be supplied for a few seconds, then vented for a few seconds, causing a pulsation of negative pressure in the sealed environment. The pulsation of the negative pressure can pulsate the nodules 902, causing further disruption of the debris 704.

In some embodiments, the cyclical application of instillation therapy and negative pressure therapy may cause micro-floating. For example, negative pressure may be applied to the sealed environment during a negative-pressure therapy cycle. Following the conclusion of the negative-pressure therapy cycle, instillation fluid may be supplied during the instillation therapy cycle. The instillation fluid may cause the contact layer 202 to float relative to the debris. As the contact layer 202 floats, it may change position relative to the position the contact layer 202 occupied during the negative-pressure therapy cycle. The position change may cause the contact layer 202 to engage a slightly different portion of the debris 704 during the next negative-pressure therapy cycle, aiding disruption of the debris 704.

The holes 210 of the contact layer 202 may generate concentrated stresses that influence disruption of the debris in different ways. For example, different shapes of the holes 210 may also focus the stresses generated by the contact layer 202 in advantageous areas. A lateral force, such as the lateral force 404, generated by a contact layer, such as the contact layer 202, may be related to a compressive force generated by applying negative pressure at a therapy pressure to a sealed therapeutic environment. For example, the lateral force 404 may be proportional to a product of a therapy pressure (TP) in the sealed environment, the compressibility factor (CF) of the contact layer 202, and a surface area (A) the second surface 208 of the contact layer 202. The relationship is expressed as follows:
Lateral force α (TP * CF * A)

In some embodiments, the therapy pressure TP is measured in N/m², the compressibility factor (CF) is dimensionless, the area (A) is measured in m², and the lateral force is measured in Newtons (N). The compressibility factor (CF) resulting from the application of negative pressure to a contact layer may be, for example, a dimensionless number that is proportional to the product of the void space percentage (VS) of a contact layer, the firmness factor (FF) of the contact layer, the strut angle (SA) of the through-holes in the contact layer, and the perforation shape factor (PSF) of the through-holes in the contact layer. The relationship is expressed as follows:
Compressibility Factor (CF) α (VS * FF * sin(SA) * PSF)

In some embodiments, the formulas described above may not precisely describe the lateral forces due to losses in force due to the transfer of the force from the contact layer to the wound. For example, the modulus and stretching of the cover 116, the modulus of the tissue site, slippage of the cover 116 over the tissue site, and friction between the contact layer 202 and the tissue site may cause the actual value of the lateral force 404 to be less than the calculated value of the lateral force 404.

Figure 10 is a perspective view of an operational step in a process for manufacturing the contact layer 202, illustrating additional details that may be associated with some embodiments. In some embodiments, a block 1002 of dressing material may be provided. For example, the dressing material of the block 1002 may be a hydrophilic reticulated polyurethane foam. Preferably, the block 1002 may be non-felted or have a firmness factor (FF) of 1. In some embodiments, the block 1002 may have a thickness 1004. The thickness 1004 can be about 30 mm.

The block 1002 can be felted to a first felting level. For example, the block 1002 can be positioned in a press 1006 having a first plate 1008 and a second plate 1010. The press 1006 can compress and heat the block 1002. In some embodiments, the press 1006 can felt the block 1002 to increase the density of the block 1002 to have a firmness factor (FF) of about 3. In other embodiments, the block 1002 may be heated before being compressed. A force applied by the press 1006 may be sufficient to decrease the thickness 1004 of the block 1002 to a thickness that is about one-third the thickness 1004 without unnecessarily blocking or closing off fluid passages through the block 1002. For example, in some embodiments, a force of about 13.8kPa (2 lbs/in² (psi)) to about (27.6kPa) 4 psi can be applied to the block 1002 by the press 1006. In some embodiments, the block 1002 may be heated before or during compression. For example, the block 1002 can be heated to a temperature greater than about 80 degrees Celsius (°C) before or during compression.

In some embodiments, the holes 210 may be formed during molding of the block 1002. In other embodiments, the holes 210 may be formed by cutting, melting, drilling, or vaporizing the block 1002 after the block 1002 is felted to the first felting level. For example, the holes 210 may be formed in the block 1002 by laser cutting the block 1002. In some embodiments, the holes 210 may be formed so that the interior surfaces of the walls 302 of the holes 210 are parallel to the thickness 212. In other embodiments, the holes 210 may be formed so that the interior surfaces of the walls 302 of the holes 210 form a non-perpendicular angle with the second surface 208. In still other embodiments, the interior surfaces of the walls 302 of the holes 210 may taper toward the center 402 of the holes 210 to form conical, pyramidal, or other irregular through-hole shapes. If the interior surfaces of the walls 302 of the holes 210 taper, the holes 210 may have a height less than the thickness 1004 of the block 1002.

Figure 11 is a perspective view of an operational step in a process for manufacturing the contact layer 202, illustrating additional details that may be associated with some embodiments. In some embodiments, the holes 210 may be formed by applying a second felting process to the block 1002. Following felting of the block 1002 to a firmness factor (FF) of about 3, the block 1002 may have a second thickness 1102. In some embodiments, the second thickness 1102 may be about 10 mm.

A second felting operation can be applied to the block 1002. The holes 210 can be formed in the contact layer 202 by using a mandrel 1104 having a surface 1106 and a plurality of shaped features or projections 1108 extending from the surface 1106. The projections 1108 can have a shape to produce a correspondingly shaped hole 210. In some embodiments, the projections 1108 may be cylindrical. In other embodiments, the projections 1108 may be conical, spherical, polygonal, or amorphous-shaped to produce a desired shape of the holes 210. The projections 1108 can be distributed on the surface 1106 in a pattern. In some embodiments, the pattern may match the desired distribution of the holes 210 in the contact layer 202 of the contact layer 202. For example, the projections 1108 can be distributed across the surface 1106 so that the projections 1108 have a strut angle and spacing of the holes 210. The projections 1108 can also have a height 1110. In some embodiments the height 1110 of the projections 1108 can be between about 5 mm and about 8 mm. In some embodiments, teach projection 1108 can have an average effective diameter between about 5 mm and about 20 mm, and in some embodiments, the average effective diameter of each projection may be about 10 mm.

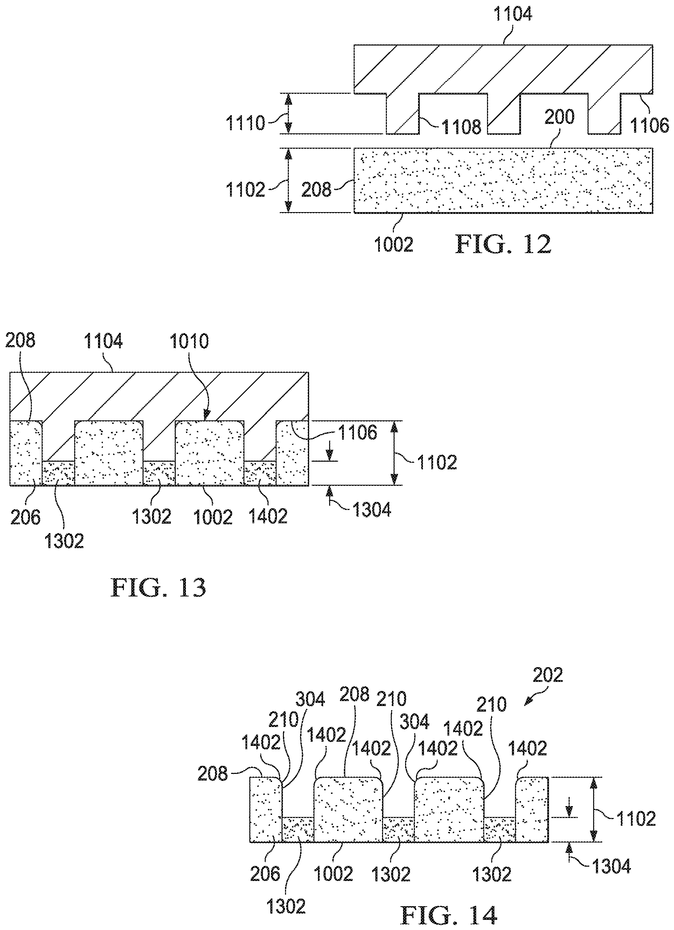

Figure 12 is a sectional view of the operational step in the process for manufacturing the contact layer 202 taken along line 11-11 of Figure 11, illustrating additional details that may be associated with some embodiments. The mandrel 1104 can be used to felt the block 1002 to a second felting level, pressing the projections 1108 into the block 1002 while heating the projections 1108 to felt the block 1002 at localized portions to a second level. The block 1002 can have the first surface 206 and the second surface 208 of the contact layer 202. The mandrel 1104 can be positioned relative to the block 1002 so that the projections 1108 and the surface 1106 are proximate to the second surface 208 of the block 1002.

Figure 13 is a sectional view of the operational step in the process for manufacturing the contact layer 202 taken along line 12-12 of Figure 11, illustrating additional details that may be associated with some embodiments. The mandrel 1104 can be moved into the block 1002. For example, the block 1002 may rest on a planar surface. The mandrel 1104 can be moved into the block 1002 so that the surface 1106 is adjacent to or contacts the second surface 208 of the block 1002. Bringing the surface 1106 of the mandrel 1104 adjacent to or into contact with the second surface 208 of the block 1002 can press the projections 1108 into the block 1002. In some embodiments, the projections 1108 can be heated while being pressed into the block 1002. For example, the projections 1108 can be heated to a temperature between 60 °C and 100 °C and, preferably, about 80 °C. Pressing of the projections 1108 into the block 1002 while the surface 1106 of the mandrel 1104 is adjacent to the second surface 208 can create zones of localized felting of the block 1002. The zones of localized felting of the block 1002 can cause localized portions 1302 of the block 1002 to be felted to a second level forming voids in the block 1002. In some embodiments, the second level can increase the density of the block 1002 at the localized portions 1302 to about five times the density of the block 1002 after the first felting process and about seven times the density of the dressing material of the block 1002 prior to the first felting process. In some embodiments, the localized portions 1302 of the block 1002 may have a thickness 1304 that is less than the second thickness 1102 of the block 1002. In some embodiments, the thickness 1304 may be between about 2 mm and about 5 mm.

Figure 14 is a sectional view of the operational step in the process for manufacturing the contact layer 202 taken along line 12-12 of Figure 11, illustrating additional details that may be associated with some embodiments. The mandrel 1104 can be removed from the block 1002. The second felting process can form the holes 210 at the localized portions 1302. The holes 210 may have a depth approximately equal to the eight 1110 of the projections 1108. In some embodiments, the second felting process can form a transition zone 1402 between the holes 210 and the second surface 208. In some embodiments, the transition zone 1402 can comprise a large radii at the perimeters 304 of the holes 210 where the second surface 208 intersects the walls 302. For example, the perimeter 304 at the transition zone 1402 can have a radius of curvature from the second surface 208 to the interior surface of the hole 210 of about 3 mm to about 5 mm. The projections 1108 of the mandrel 1104 may not heat the transition zone 1402 to a temperature that is as high as the temperature applied to the localized portions 1302. The projections 1108 of the mandrel 1104 also apply compression vertically to the second surface 208, providing a transition of heat and force at the transition zones 1402 to create a radius of curvature in response to the non-uniformly applied heat and force. In some embodiments, the transition zones 1502 can prevent sharp edge in-growth into the thinner regions of the contact layer 202, which can happen with the non-felted, cut designs. A non-felted foam thins as it approaches the point of the cut edges, allowing ingrowth and reducing the local tensile strength in these regions which may make it more likely that the foam separates when the dressing is removed.

Formation of the holes 210 may thermoform the material of the contact layer 202, for example a compressed foam or a felted foam, causing the interior surface of the walls 302 to be smooth. As used herein, smoothness may refer to the formation of the holes 210 that causes the interior surface of the walls 302 that extends into the contact layer 202 from the second surface 208 to be substantially free of pores if compared to a non-thermoformed portion of the contact layer 202. For example, the felting of the block 1002 by the mandrel 1104 and the projections 1108 may close any pores on the interior surfaces of the walls 302. In some embodiments, a smooth interior surface of the walls 302 may further limit or otherwise inhibit ingrowth of tissue into the contact layer 202 through the holes 210.

The systems, apparatuses, and methods described herein may provide significant advantages. For example, the method of manufacturing the contact layer 202 may provide a single piece design having a simplified manufacturing process. The manufacturing process can also reduce the risk of formation of particulates that may contaminate a pouch or tissue site. The manufacturing process increases the uniformity of the contact layer 202 by better ensuring that each contact cavity has a same or desired depth. The manufacturing process also creates edges having a radius of curvature, eliminating a thin edge that may suffer from in-growth and have a lower tensile strength at separation from a tissue site.

## Claims

1. A dressing for treating a tissue site, the dressing comprising a tissue interface (202) having:
a first side (208), a second side (206), and a first thickness (212, 1102) from the first side (208) to the second side (206);
a plurality of blind apertures (210) disposed in the first side (208), each of the blind apertures having a second thickness (1304) from the first side to the second side (206); and
the tissue interface having a first density at the first thickness (212, 1102) and a second density at the second thickness (1304);
wherein the second thickness (1304) is less than the first thickness (212, 1102), and the second density is higher than the first density.

2. The dressing of claim 1, wherein the second density is five times greater than the first density.

3. The dressing of claim 16, wherein the first thickness is about 10 mm.

4. The dressing of claim 16, wherein the second thickness is between about 2 mm and about 4 mm.

5. The dressing of claim 16, further comprising transition zones (1402) between the first thickness (212, 1102) and the second thickness (1304).

6. The dressing of claim 22, wherein the transition zones (1402) comprise large radii.

7. The dressing of claim 16, wherein the tissue interface comprises an open-cell reticulated foam.

8. A system for providing negative-pressure therapy to a tissue site, the system comprising:
a tissue interface according to any preceding claim;
a sealing member configured to be disposed over the tissue interface to create a sealed space; and
a negative-pressure source configure to be fluidly coupled to the sealed space.

## Patentansprüche

1. Ein Verband zur Behandlung einer Gewebestelle, wobei der Verband eine Gewebeschnittstelle (202) umfasst, die aufweist:
eine erste Seite (208), eine zweite Seite (206) und eine erste Dicke (212, 1102) von der ersten Seite (208) zu der zweiten Seite (206);
eine Vielzahl von Blindöffnungen (210), die in der ersten Seite (208) angeordnet sind, wobei jede der Blindöffnungen eine zweite Dicke (1304) von der ersten Seite zu der zweiten Seite (206) aufweist; und
wobei die Gewebeschnittstelle eine erste Dichte bei der ersten Dicke (212, 1102) und eine zweite Dichte bei der zweiten Dicke (1304) aufweist;
wobei die zweite Dicke (1304) geringer ist als die erste Dicke (212, 1102) und die zweite Dichte höher ist als die erste Dichte.

2. Der Verband nach Anspruch 1, wobei die zweite Dichte fünfmal größer ist als die erste Dichte.

3. Der Verband nach Anspruch 16, wobei die erste Dicke etwa 10 mm beträgt.

4. Der Verband nach Anspruch 16, wobei die zweite Dicke zwischen etwa 2 mm und etwa 4 mm beträgt.

5. Der Verband nach Anspruch 16, der ferner Übergangszonen (1402) zwischen der ersten Dicke (212, 1102) und der zweiten Dicke (1304) aufweist.

6. Der Verband nach Anspruch 22, wobei die Übergangszonen (1402) große Radien aufweisen.

7. Der Verband nach Anspruch 16, wobei die Gewebegrenzfläche einen offenzelligen retikulierten Schaumstoff umfasst.

8. Ein System zum Bereitstellen einer Unterdrucktherapie an einer Gewebestelle, wobei das System aufweist:
eine Gewebegrenzfläche nach einem der vorstehenden Ansprüche;
ein Dichtungselement, das so konfiguriert ist, dass es über der Gewebegrenzfläche angeordnet wird, um einen abgedichteten Raum zu erzeugen; und eine Unterdruckquelle, die so konfiguriert ist, dass sie mit dem abgedichteten Raum in Fluidverbindung steht.

## Revendications

1. Pansement pour le traitement d'un site tissulaire, le pansement comprenant une interface tissulaire (202) ayant :
un premier côté (208), un second côté (206) et une première épaisseur (212, 1102) entre le premier côté (208) et le second côté (206) ;
une pluralité d'ouvertures aveugles (210) disposées dans le premier côté (208), chacune des ouvertures aveugles ayant une seconde épaisseur (1304) du premier côté au second côté (206) ; et
l'interface tissulaire a une première densité à la première épaisseur (212, 1102) et une seconde densité à la seconde épaisseur (1304) ;
dans lequel la seconde épaisseur (1304) est inférieure à la première épaisseur (212, 1102), et la seconde densité est supérieure à la première densité.

2. Pansement selon la revendication 1, dans lequel la seconde densité est cinq fois supérieure à la première densité.

3. Pansement selon la revendication 16, dans lequel la première épaisseur est d'environ 10 mm.

4. Pansement selon la revendication 16, dans lequel la seconde épaisseur est comprise entre environ 2 mm et environ 4 mm.

5. Pansement selon la revendication 16, comprenant en outre des zones de transition (1402) entre la première épaisseur (212, 1102) et la seconde épaisseur (1304).

6. Pansement selon la revendication 22, dans lequel les zones de transition (1402) ont des rayons importants.

7. Pansement selon la revendication 16, dans lequel l'interface tissulaire comprend une mousse réticulée à cellules ouvertes.

8. Système permettant d'administrer une thérapie par pression négative à un site tissulaire, le système comprenant :
une interface tissulaire selon l'une quelconque revendication précédente ;
un élément de scellement conçu pour être disposé sur l'interface tissulaire afin de créer un espace scellé ; et une source de pression négative conçue pour être accouplée fluidiquement à l'espace scellé.
